(19) Europäisches Patentamt / European Patent Office / Office européen des brevets

(11) **EP 3 937 074 A1**

(12) **EUROPEAN PATENT APPLICATION**
published in accordance with Art. 153(4) EPC

(43) Date of publication:
**12.01.2022 Bulletin 2022/02**

(21) Application number: **20795577.4**

(22) Date of filing: **20.04.2020**

(51) Int Cl.:
**G06K 9/00** *(2022.01)*

(86) International application number:
**PCT/CN2020/085509**

(87) International publication number:
**WO 2020/216158 (29.10.2020 Gazette 2020/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2019 CN 201910343291**

(71) Applicant: **Huawei Technologies Co., Ltd.**
**Shenzhen, Guangdong 518129 (CN)**

(72) Inventors:
• **ZHANG, Xiaoping**
 **Shenzhen, Guangdong 518129 (CN)**
• **WU, Huangwei**
 **Shenzhen, Guangdong 518129 (CN)**

(74) Representative: **Gill Jennings & Every LLP**
**The Broadgate Tower**
**20 Primrose Street**
**London EC2A 2ES (GB)**

(54) **METHOD AND APPARATUS FOR BLOOD PRESSURE MEASUREMENT PROCESSING, AND ELECTRONIC DEVICE**

(57) A method and an apparatus for processing blood pressure measurement, and an electronic device are provided. The method includes: obtaining a facial feature point of a to-be-measured object (S101); determining a first height difference between the facial feature point and an origin of a second coordinate system based on a first coordinate of the facial feature point in the second coordinate system and a first attitude angle of the second coordinate system relative to a first coordinate system, where a preset point on an electronic device is used as the origin of the second coordinate system (S102); determining a third height difference between the origin of the second coordinate system and the heart of the object based on the first height difference and a second height difference, where the second height difference is a height difference between the facial feature point and the heart of the object (S103); and determining blood pressure of the object based on a blood pressure measurement signal of the object collected by a blood pressure measurement signal collection apparatus and the third height difference (S104). The method can greatly improve usability of a blood pressure measurement device.

Obtain a facial feature point of a to-be-measured object — S101

Determine a first height difference between the facial feature point and an origin of a second coordinate system based on a first coordinate of the facial feature point in the second coordinate system and a first attitude angle of the second coordinate system relative to a first coordinate system, where a preset point on an electronic device is used as the origin of the second coordinate system — S102

Determine a third height difference between the origin of the second coordinate system and a heart of the object based on the first height difference and a second height difference, where the second height difference is a height difference between the facial feature point and the heart of the object — S103

Determine blood pressure of the object based on a blood pressure measurement signal of the object collected by a blood pressure measurement signal collection apparatus and the third height difference — S104

FIG. 1

EP 3 937 074 A1

**Description**

This application claims priority to Chinese Patent Application No. 201910343291.7, filed with the China National Intellectual Property Administration on April 26, 2019 and entitled "METHOD AND APPARATUS FOR PROCESSING BLOOD PRESSURE MEASUREMENT, AND ELECTRONIC DEVICE", which is incorporated herein by reference in its entirety.

**TECHNICAL FIELD**

Embodiments of this application relate to intelligent device technologies, and in particular, to a method and an apparatus for processing blood pressure measurement, and an electronic device.

**BACKGROUND**

Blood pressure is lateral pressure of blood on a vascular wall in a unit area when the blood flows in blood vessels. A blood pressure value is an important indicator for measuring a human health condition. Therefore, how to perform blood pressure measurement to provide an accurate blood pressure measurement result is of great significance. Blood pressure measurement methods may be divided into two types: a direct measurement method and an indirect measurement method. The direct measurement method is to percutaneously deliver a long catheter with melted anticoagulants to an aorta, where the catheter is connected to a pressure sensor to directly display blood pressure. The indirect measurement method is mainly to measure blood pressure by using a blood pressure measurement device. Compared with the direct measurement method, the indirect measurement method has advantages of easiness and convenience for operation, and therefore is widely used in various scenarios in which blood pressure measurement needs to be performed. After blood pressure measurement devices have undergone development processes of mercury sphygmomanometers, upper-arm electronic sphygmomanometers, wrist electronic sphygmomanometers, and the like, with continuous development of mobile terminal technologies such as mobile phones, blood pressure measurement devices such as watches and mobile phones with blood pressure measurement functions appear. In this way, a blood pressure measurement technology continuously develops toward directions of portability and miniaturization in combination with consumer electronics.

A mobile phone with a blood pressure measurement function is used as an example. In a conventional technology, when measuring blood pressure by using the mobile phone, a user is required to hold the mobile phone and collect a signal of a specific measured part on a body of the user by using a related sensor on the mobile phone. After collection is performed for a period of time, the mobile phone calculates a blood pressure value based on the collected signal. When blood pressure is collected, it is required that a height of the measured part is kept consistent with that of the heart of the user. Otherwise, a relatively large error may be caused to a measurement result.

However, when the method in the conventional technology is used, usability of a blood pressure measurement device may be relatively low. This further leads to poor user experience.

**SUMMARY**

Embodiments of this application provide a method and an apparatus for processing blood pressure measurement, and an electronic device, so that usability of a blood pressure measurement device can be greatly improved.

A first aspect of the embodiments of this application provides a method for processing blood pressure measurement. In this method, an electronic device obtains a facial feature point of a to-be-measured object; determines a first height difference between the facial feature point and an origin of a second coordinate system based on a first coordinate of the facial feature point in the second coordinate system and a first attitude angle of the second coordinate system relative to a first coordinate system, where a preset point on the electronic device is used as the origin of the second coordinate system; then determines a third height difference between the origin of the second coordinate system and the heart of the object based on the first height difference and a second height difference, where the second height difference is a height difference between the facial feature point and the heart of the object; and further determines blood pressure of the object based on a blood pressure measurement signal of the object collected by a blood pressure measurement signal collection apparatus and the third height difference.

According to the method provided in this embodiment of this application, the facial feature point of the to-be-measured object is obtained, a second coordinate of the facial feature point in the first coordinate system may be obtained based on the first coordinate of the facial feature point in the second coordinate system and the first attitude angle of the second coordinate system relative to the first coordinate system, and the third height difference between the origin of the second coordinate system and the heart of the to-be-measured object may be obtained based on the first height difference that is between the facial feature point and the origin of the second coordinate system and that is identified by the second coordinate, and the second height difference between the facial feature point and the heart of the to-be-measured object. Because a measured part of the to-be-measured object is in contact with the electronic device during blood pressure measurement, a height of the origin of the second coordinate system may be kept consistent with that of the measured part. Correspondingly, the third height difference may represent a height difference between the measured part and the heart. The height difference is exactly a height difference that causes a blood pressure measurement error. Therefore, corresponding compensation processing is further performed based on the third height difference and the signal collected by the blood pressure measurement signal collection apparatus, so that impact of the height difference on a blood pressure measurement result can be eliminated, thereby obtaining an accurate blood pressure measurement result. Therefore, in this embodiment, even if the height of the measured part is inconsistent with that of the heart of the to-be-measured object, an accurate measurement result can be obtained. Therefore, this embodiment can greatly improve usability of a blood pressure measurement device, thereby greatly improving user experience. Moreover, for an electronic device with relatively high space flexibility for a measured part, such as a mobile phone or a watch, after this embodiment is used, because a requirement that the height of the measured part is kept consistent with that of the heart of the user no longer needs to be satisfied, improvement in usability is particularly evident.

In a possible design, before the first height difference between the facial feature point and the origin of the second coordinate system is determined based on the first coordinate of the facial feature point in the second coordinate system and the first attitude angle of the second coordinate system relative to the first coordinate system, the first coordinate of the facial feature point in the second coordinate system may be determined based on a coordinate of the facial feature point in a fourth coordinate system of the electronic device, where a coordinate axis of the fourth coordinate system is collinear with an optical axis, and there is a preset translation amount between the fourth coordinate system and the second coordinate system.

In this possible design, when the facial feature point is a middle point between pupil centers of two eyes of the object, the first coordinate of the facial feature point in the second coordinate system may be determined by using the following process:

collecting a facial image of the to-be-measured object; determining the coordinate of the facial feature point in the fourth coordinate system based on pixel positions of the pupil centers of the two eyes of the to-be-measured object in the facial image; and determining the first coordinate of the facial feature point in the second coordinate system based on the coordinate of the facial feature point in the fourth coordinate system and the translation amount between the fourth coordinate system and the second coordinate system.

In a possible design, before the third height difference between the origin of the second coordinate system and the heart of the object is determined based on the second coordinate and the second height difference, the second height difference may further be first determined.

A method for determining the second height difference may include the following three manners:

In a first manner, there is a preset correspondence between the second height difference and at least one parameter.

In a second manner, configuration parameter information entered by the object is received, where the configuration parameter information includes the second height difference.

In a third manner, a third attitude angle of a third coordinate system relative to the first coordinate system may be determined based on a second attitude angle of the third coordinate system relative to the second coordinate system and the first attitude angle, where the facial feature point is used as an origin of the third coordinate system; then a fourth attitude angle is determined based on a mapping relationship between the third attitude angle and the fourth attitude angle, where the fourth attitude angle is an attitude angle of the torso of the to-be-measured object relative to the first coordinate system; and then the second height difference is determined based on the third attitude angle and the fourth attitude angle.

In the foregoing third manner, when the second height difference is determined based on the third attitude angle and the fourth attitude angle, the second height difference may be determined based on the third attitude angle, the fourth attitude angle, a preset height difference, and preset ratio information, where the preset height difference is a height difference between the facial feature point and the heart of the object when the torso and the head of the object are vertical, and the preset ratio information is a ratio of a height difference between the facial feature point and a neck preset point of the object to the preset height difference when the torso and the head of the object are vertical. Alternatively, the second height difference may be determined based on the third attitude angle, the fourth attitude angle, a height difference between the facial feature point and a neck preset point, and a height difference between the neck preset point and the heart.

An accurate second height difference can be obtained by using any one of the foregoing three manners.

In a possible design, when the blood pressure of the object is determined based on the blood pressure measurement signal of the object collected by the blood pressure measurement signal collection apparatus and the third height difference, a blood pressure compensation value may be determined based on the third height difference; and then the blood pressure of the object is determined based on the blood pressure measurement signal of the object collected by the blood pressure measurement signal collection apparatus and the blood pressure compensation value.

After the blood pressure compensation value obtained based on the third height difference is applied to the blood pressure measurement signal collected by the blood pressure measurement signal collection apparatus, a measurement error generated by a height difference between the measured part and the heart can be greatly reduced.

In a possible design, the blood pressure compensation value may be determined based on the third height difference and a preset blood pressure compensation model, where the blood pressure compensation model is used for representing a correspondence between the third height difference and the blood pressure compensation value.

In a possible design, before the blood pressure compensation value is determined based on the third height difference and the preset blood pressure compensation model, the method further includes:

collecting blood pressure error information under a plurality of third height differences; and
establishing the blood pressure compensation model based on the blood pressure error information under the plurality of third height differences.

In a possible design, whether a height of the electronic device changes when the blood pressure measurement signal collection apparatus collects the blood pressure measurement signal may further be detected, and collecting the blood pressure measurement signal is stopped when the height of the electronic device changes.

A second aspect of the embodiments of this application provides an apparatus for processing blood pressure measurement. The apparatus includes a processing module, where the processing module is configured to: obtain a facial feature point of a to-be-measured object; determine a first height difference between the facial feature point and an origin of a second coordinate system based on a first coordinate of the facial feature point in the second coordinate system and a first attitude angle of the second coordinate system relative to a first coordinate system, where a preset point on an electronic device is used as the origin of the second coordinate system; determine a third height difference between the origin of the second coordinate system and the heart of the object based on the first height difference and a second height difference, where the second height difference is a height difference between the facial feature point and the heart of the object; and determine blood pressure of the object based on a blood pressure measurement signal of the object collected by a blood pressure measurement signal collection apparatus and the third height difference.

In a possible design, the processing module is further configured to:
determine the first coordinate of the facial feature point in the second coordinate system based on a coordinate of the facial feature point in a fourth coordinate system of the electronic device, where a coordinate axis of the fourth coordinate system is collinear with an optical axis, and there is a preset translation amount between the fourth coordinate system and the second coordinate system.

In a possible design, when the facial feature point is a middle point between pupil centers of two eyes of the object, the processing module may be specifically configured to:

collect a facial image of the to-be-measured object;
determine the coordinate of the facial feature point in the fourth coordinate system based on pixel positions of the pupil centers of the two eyes of the to-be-measured object in the facial image; and
determine the first coordinate of the facial feature point in the second coordinate system based on the coordinate of the facial feature point in the fourth coordinate system and the translation amount between the fourth coordinate system and the second coordinate system.

In a possible design, the processing module is further configured to:
determine the second height difference.

In a possible design, there is a preset correspondence between the second height difference and at least one parameter.

In a possible design, the processing module is specifically configured to:
receive configuration parameter information entered by the object, where the configuration parameter information includes the second height difference.

In a possible design, the processing module is specifically configured to:

determine a third attitude angle of a third coordinate system relative to the first coordinate system based on a second attitude angle of the third coordinate system relative to the second coordinate system and the first attitude angle, where the facial feature point is used as an origin of the third coordinate system;
determine a fourth attitude angle based on a mapping relationship between the third attitude angle and the fourth attitude angle, where the fourth attitude angle is an attitude angle of the torso of the to-be-measured object relative to the first coordinate system; and
determine the second height difference based on the third attitude angle and the fourth attitude angle.

In a possible design, the processing module is specifically configured to:

determine the second height difference based on the third attitude angle, the fourth attitude angle, a preset height difference, and preset ratio information, where
the preset height difference is a height difference between the facial feature point and the heart of the object when the torso and the head of the object are vertical, and the preset ratio information is a ratio of a height difference between the facial feature point and a neck preset point of the object to the preset height difference when the torso and the head of the object are vertical.

In a possible design, the processing module is specifically configured to:
determine the second height difference based on the third attitude angle, the fourth attitude angle, a height difference between the facial feature point and a neck preset point, and a height difference between the neck preset point and the heart.

In a possible design, the processing module is specifically configured to:

determine a blood pressure compensation value based on the third height difference; and
determine the blood pressure of the object based on the blood pressure measurement signal of the object collected by the blood pressure measurement signal collection apparatus and the blood pressure compensation value.

In a possible design, the processing module is specifically configured to:
determine the blood pressure compensation value based on the third height difference and a preset blood pressure compensation model, where the blood pressure compensation model is used for representing a correspondence between the third height difference and the blood pressure compensation value.

In a possible design, the processing module is further configured to:

collect blood pressure error information under a plurality of third height differences; and
establish the blood pressure compensation model based on the blood pressure error information under the plurality of third height differences.

In a possible design, the processing module is further configured to:
detect whether a height of the electronic device changes when the blood pressure measurement signal collection apparatus collects the blood pressure measurement signal, and stop collecting the blood pressure measurement signal when the height of the electronic device changes.

For beneficial effects of the apparatus provided in the second aspect and the possible implementations of the second aspect, refer to beneficial effects brought by the first aspect and the possible implementations of the first aspect. Details are not described herein again.

A third aspect of the embodiments of this application provides an electronic device. The electronic device includes a processor and a memory, where the memory is configured to store computer-executable program code, the program code includes instructions, and when the processor executes the instructions, the instructions enable the network device to perform the method according to the first aspect or the possible implementations of the first aspect.

According to a fourth aspect, an embodiment of this application provides a computer program product including instructions. When the computer program product runs on a computer, the computer is enabled to perform the method according to the first aspect or the possible implementations of the first aspect.

According to a fifth aspect, an embodiment of this application provides a computer-readable storage medium. The computer-readable storage medium stores instructions. When the instructions run on a computer, the computer is enabled to perform the method according to the first aspect or the possible implementations of the first aspect.

## BRIEF DESCRIPTION OF DRAWINGS

FIG. 1 is a schematic flowchart of a blood pressure processing method according to an embodiment of this application;
FIG. 2 is a schematic diagram of a coordinate and height differences when blood pressure is measured by using a mobile phone;
FIG. 3 is a schematic diagram of a fourth coordinate system and a second coordinate system;
FIG. 4 is a schematic flowchart of a blood pressure processing method according to an embodiment of this application;
FIG. 5 is a schematic diagram of imaging of a human face in a fourth coordinate system;
FIG. 6 is a schematic diagram of a posture in which a torso and a head are perpendicular to a ground;

FIG. 7(a), FIG. 7(b), and FIG. 7(c) are respectively schematic diagrams of three measurement postures;
FIG. 8 is a schematic flowchart of a blood pressure processing method according to an embodiment of this application;
FIG. 9 is a schematic diagram of estimating a third attitude angle;
FIG. 10 shows an example of selecting a plurality of facial feature points;
FIG. 11 is a schematic flowchart of a blood pressure processing method according to an embodiment of this application;
FIG. 12 is a schematic structural diagram of an apparatus for processing blood pressure measurement according to an embodiment of this application; and
FIG. 13 is a schematic structural diagram of an electronic device 1300 according to an embodiment of this application.

## DESCRIPTION OF EMBODIMENTS

When a blood pressure measurement device calculates a blood pressure value by collecting a signal of a measured part, a height change of the measured part has relatively large impact on a blood pressure measurement result. To alleviate the impact of the height change of the measured part on the blood pressure measurement result, in a conventional technology, when a user measures blood pressure, it is required that a height of the measured part is kept consistent with that of the heart of the user. For example, when measuring blood pressure by using a mobile phone, the user is required to hold the mobile phone and collect a signal of a specific measured part on the body of the user by using a related sensor on the mobile phone. After collection is performed for a period of time, the mobile phone calculates a blood pressure value based on the collected signal. When blood pressure is collected, it is required that the height of the measured part is kept consistent with that of the heart of the user. Compared with a conventional upper-arm sphygmomanometer, when blood pressure is measured by using an electronic device such as a mobile phone or a watch, space flexibility of the measured part is higher. Therefore, it is more difficult to keep the height of the measured part consistent with that of the heart of the user. Therefore, when the method in the conventional technology is used, usability of the blood pressure measurement device may be relatively low. This further leads to poor user experience. In addition, because it is relatively difficult to keep the height of the measured part consistent with that of the heart of the user, when the method in the conventional technology is used, accuracy of a measurement result is also likely to be low.

The technical solutions provided in the embodiments of this application are intended to resolve the foregoing problem.

The embodiments of this application may be applied to a scenario in which an electronic device is used to measure blood pressure of a to-be-measured object. The electronic device may be any electronic device that has a blood pressure measurement function, for example, a mobile phone, a watch, or a band. A specific form of the electronic device is not limited in the embodiments of this application. The electronic device collects a blood pressure signal by using a blood pressure measurement signal collection apparatus. The blood pressure measurement signal collection apparatus may be a built-in apparatus of the electronic device, or may be an independent external apparatus, and a communication connection is established between the apparatus and the electronic device. The blood pressure measurement signal collection apparatus may be, for example, a blood pressure measurement signal collection sensor. A specific form and a position of the blood pressure measurement signal collection apparatus are not limited in the embodiments of this application. When blood pressure measurement is performed for the to-be-measured object, a contact manner between a measured part of the to-be-measured object and the blood pressure measurement signal collection apparatus may be a manner such as touch or wearing. The contact manner is not specifically limited in the embodiments of this application. In addition, in the embodiments of this application, a camera is disposed on the electronic device, to collect a facial image of the to-be-measured object. The camera may be a front-facing camera, or may be a rear-facing camera. A position of the camera is not specifically limited in the embodiments of this application. Moreover, the camera may be a monocular camera or a multi-view camera, and the camera may use a time of flight (time of flight, ToF) technology, a structured light technology, an RGB color (red, green, blue, RGB) binocular technology, and the like. A quantity and technical structures of cameras are not specifically limited in the embodiments of this application.

In addition, the to-be-measured object to which the embodiments of this application are applicable may be a person, but the embodiments of this application are not limited thereto. The measured part of the to-be-measured object may be a finger, a wrist, an arm, or the like. This is not specifically limited in the embodiments of this application.

To enable a person skilled in the art to better understand the embodiments of this application, the following first explains technical terms used in the embodiments of this application.

1. First coordinate system

An origin of the first coordinate system is an arbitrarily specified spatial point, three coordinate axes of the first coordinate system are perpendicular to each other, and one of the coordinate axes is vertically upward (a sky direction for short) or downward (a ground direction for short).

It should be noted that, in the embodiments of this application, the other two coordinate axes different from the vertically upward or downward coordinate axis in the first coordinate system may point to any direction in a horizontal plane according to a specified angle.

In an example, one coordinate axis of the first coordinate system points to the sky direction, another coordinate axis points to an east direction, and the rest coordinate axis points to a north direction.

In another example, one coordinate axis of the first coordinate system points to the ground direction, another coordinate axis points to a west direction, and the rest coordinate axis points to a south direction.

In the following embodiments of this application, an example in which the first coordinate system is the coordinate system in the first example is used for description.

2. Second coordinate system

A particular specified point on the electronic device is used as an origin of the second coordinate system, a first coordinate axis points to a direction of the head of the electronic device, a second coordinate axis points to a screen side of the electronic device, is perpendicular to a screen, and is outward, and a third coordinate axis is perpendicular to the first coordinate axis and the second coordinate axis.

3. Third coordinate system

The third coordinate system is a coordinate system corresponding to the face of the to-be-measured object, and one coordinate axis of the third coordinate system is perpendicular to a plane formed by a plurality of feature points of the face. The other two coordinate axes of the third coordinate system are perpendicular to the coordinate axis.

4. Fourth coordinate system

The fourth coordinate system is a coordinate system corresponding to a camera that is disposed on the electronic device and that is configured to collect a facial image, and one coordinate axis of the fourth coordinate system is collinear with an optical axis of the camera. The other two coordinate axes of the fourth coordinate system are perpendicular to the coordinate axis.

Optionally, the three coordinate axes of the fourth coordinate system may be respectively in a same direction as the three coordinate axes of the second coordinate system.

The following describes the technical solutions in the embodiments of this application in detail with reference to specific embodiments. The following several specific embodiments may be combined with each other. Same or similar concepts or processes may not be described in some embodiments.

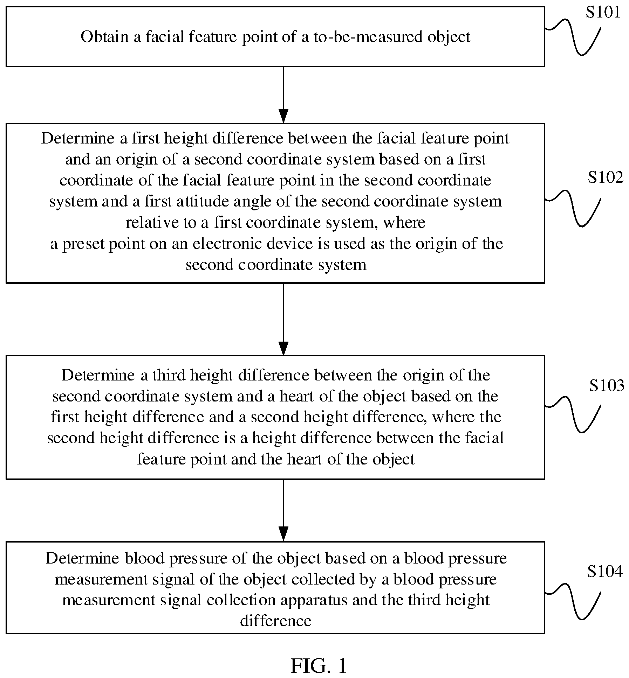

FIG. 1 is a schematic flowchart of a blood pressure processing method according to an embodiment of this application. An execution body of the method is the foregoing electronic device. For ease of description, the following embodiment of this application is described by using a mobile phone as an example. As shown in FIG. 1, the method includes the following steps.

S101: Obtain a facial feature point of a to-be-measured object.

Optionally, if the to-be-measured object is a person, the facial feature point is a human facial feature point. If the to-be-measured object is another creature, such as a mammal, the facial feature point is a feature point of the face of an animal.

The facial feature point may refer to a specific position of the face. For example, the pupil center of the left eye, the pupil center of the right eye, the middle point of the lower lip, and the middle point between the pupil centers of two eyes may all be used as facial feature points. In this embodiment of this application, one facial feature point may be used, or a plurality of facial feature points may be used.

Optionally, after being started, the electronic device may automatically obtain the facial feature point of the to-be-measured object, or may obtain the facial feature point of the to-be-measured object under a specific trigger condition.

For example, when the electronic device is a mobile phone, a front-facing camera may be disposed on the mobile phone. After a user holding the mobile phone taps an icon of a blood pressure measurement application (application, APP) or taps a specific button for starting blood pressure measurement on the mobile phone, the mobile phone receives a blood pressure measurement indication of the user for the to-be-measured object. In this case, the mobile phone starts the front-facing camera, and the front-facing camera collects a facial image of the to-be-measured object, and extracts the facial feature point from the facial image. In this process, the mobile phone may determine whether the front-facing camera collects facial information required for blood pressure measurement and successfully extracts the preset facial feature point. If the front-facing camera collects the facial information required for blood pressure measurement and successfully extracts the preset facial feature point, the following steps continue to be performed; or if the front-facing camera fails to collect the facial information required for blood pressure measurement or extract the preset facial feature point, the mobile phone may prompt, in a form of voice or a text, the user to adjust an angle of the mobile phone, so that the front-facing camera can collect complete facial information and extract the facial feature point.

In the foregoing example, the user holding the mobile phone and the to-be-measured object may be a same person, or may be different persons.

S102: Determine a first height difference between the facial feature point and an origin of a second coordinate system based on a first coordinate of the facial feature point in the second coordinate system and a first attitude angle of the second coordinate system relative to a first coordinate system, where a preset point on the electronic device is used as the origin of the second coordinate system.

For example, the coordinate point on the electronic device may be a screen center point of the electronic device, or may be a center point of a blood pressure measurement signal collection apparatus on the electronic device. The center point may be specifically a center point of a region that is in contact with a measured part of the to-be-measured object and that is in the blood pressure measurement signal collection apparatus.

In an optional implementation, an origin of the first coordinate system may overlap the origin of the second coordinate system. In this case, a coordinate value of the facial feature point on a coordinate axis pointing to a sky direction or a ground direction in the first coordinate system may be obtained based on the first coordinate and the first attitude angle, where the coordinate value is the first height difference.

In another optional implementation, the origin of the first coordinate system may not overlap the origin of the second coordinate system. In this case, a height difference between the origin of the first coordinate system and the origin of the second coordinate system may be first obtained, and after the coordinate value of the facial feature point on the coordinate axis pointing to the sky direction or the ground direction in the first coordinate system is obtained based on the first coordinate and the first attitude angle, the foregoing origin height difference is eliminated based on the coordinate value, to obtain the first height difference. The height difference between the origin of the first coordinate system and the origin of the second coordinate system may be a preset height difference, or may be a height difference obtained through measurement after the origin of the first coordinate system is selected by the user. This is not specifically limited in this embodiment of this application.

A process of determining the first height difference is described below by using an example in which the origin of the first coordinate system overlaps the origin of the second coordinate system.

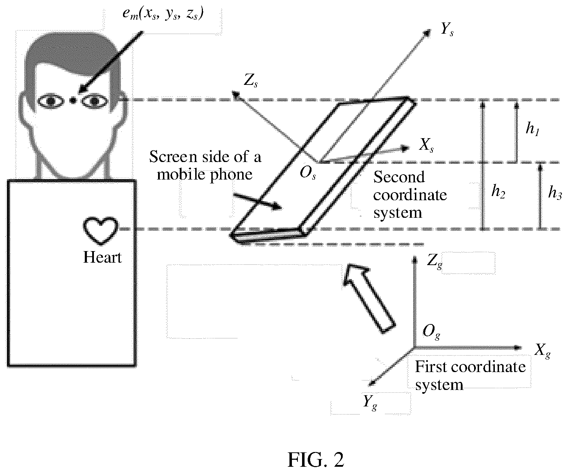

FIG. 2 is a schematic diagram of a coordinate and height differences when blood pressure is measured by using a mobile phone. It should be noted that, to clearly distinguish the first coordinate system from the second coordinate system, the origin of the first coordinate system and the origin of the second coordinate system shown in FIG. 2 are not located at a same position, but actually, the origins overlap. As shown in FIG. 2, the to-be-measured object is a person, the facial feature point $e_m$ is a middle point between pupil centers of two eyes of the person, the origin $Os$ of the second coordinate system $O_sX_sY_sZ_s$ is a screen center point of the mobile phone, a coordinate axis $Ys$ points to a direction of the head of the mobile phone, a coordinate axis $Zs$ is perpendicular to a screen on a screen side of the mobile phone and is outward, and a coordinate axis $Xs$ is perpendicular to the coordinate axis $Ys$ and the coordinate axis $Zs$. $(x_s, y_s, z_s)$ is the first coordinate of $e_m$ in the second coordinate system $O_sX_sY_sZ_s$. In addition, in FIG. 2, $O_gX_gY_gZ_g$ represents the first coordinate system in which a spatial position of an origin can be arbitrarily specified, where $O_g$ is the origin of the coordinate system, $X_g$ points to the due east direction, $Y_g$ points to the due north direction, and $Z_g$ is upward perpendicular to a horizontal plane. It can be learned from FIG. 2 that there is a particular attitude angle of the second coordinate system relative to the first coordinate system, and the origin of the second coordinate system overlaps the origin of the first coordinate system. Therefore, based on the first coordinate $(x_s, y_s, z_s)$ of the facial feature point $e_m$ in the second coordinate system and the first attitude angle of the second coordinate system relative to the first coordinate system, a second coordinate of the facial feature point $e_m$ in the first coordinate system can be obtained. A coordinate value of the second coordinate in a direction of the axis $Z_g$ is equal to a height difference between the facial feature point and the origin of the first coordinate system. Because the origin of the second coordinate system overlaps the origin of the first coordinate system, the coordinate value is also equal to the first height difference between the facial feature point and the origin of the second coordinate system. In this embodiment of this application, the first height difference is denoted as $h_1$.

S103: Determine a third height difference between the origin of the second coordinate system and the heart of the object based on the first height difference and a second height difference, where the second height difference is a height difference between the facial feature point and the heart of the object.

In addition, refer to FIG. 2. There is a second height difference between the facial feature point $e_m$ and the heart of the to-be-measured object. In this embodiment of this application, the second height difference is denoted as $h_2$.

In a specific implementation process, the measured part of the to-be-measured object is in contact with the electronic device, and spatial positions of the origins of the second coordinate system and the first coordinate system relative to the mobile phone may be freely specified and fixed. Therefore, a height of the origin of the second coordinate system may be kept the same as that of the measured part. Therefore, a height difference between the origin of the electronic device and the heart of the to-be-measured object is the third height difference between the measured part and the heart. In this embodiment of this application, the third height difference is denoted as $h_3$. An error during blood pressure measurement is generated by the third height difference $h_3$.

Refer to FIG. 2. It can be learned that, after the first height difference $h_1$ and the second height difference $h_2$ are obtained in the foregoing steps, a difference between the second height difference $h_2$ and the first height difference $h_1$ is calculated, and the obtained difference is the third height difference $h_3$.

S104: Determine blood pressure of the object based on a blood pressure measurement signal of the object collected by the blood pressure measurement signal collection apparatus and the third height difference.

Optionally, based on the blood pressure measurement signal collected by the blood pressure measurement signal collection apparatus, the collected blood pressure measurement signal is processed by using a specific blood pressure measurement algorithm, to obtain original blood pressure, and on this basis, the original blood pressure is compensated based on the third height difference, to obtain an accurate blood pressure value of the to-be-measured object.

In this embodiment, the facial feature point of the to-be-measured object is obtained, a second coordinate of the facial feature point in the first coordinate system may be obtained based on the first coordinate of the facial feature point in the second coordinate system and the first attitude angle of the second coordinate system relative to the first coordinate system, and the third height difference between the origin of the second coordinate system and the heart of the to-be-measured object may be obtained based on the first height difference that is between the facial feature point and the origin of the second coordinate system and that is identified by the second coordinate, and the second height difference between the facial feature point and the heart of the to-be-measured object. Because a measured part of the to-be-measured object is in contact with the electronic device during blood pressure measurement, a height of the origin of the second coordinate system may be kept consistent with that of the measured part. Correspondingly, the third height difference may represent a height difference between the measured part and the heart. The height difference is exactly a height difference that causes a blood pressure measurement error. Therefore, corresponding compensation processing is further performed based on the third height difference and the signal collected by the blood pressure measurement signal collection apparatus, so that impact of the height difference on a blood pressure measurement result can be eliminated, thereby obtaining an accurate blood pressure measurement result. Therefore, in this embodiment, even if the height of the measured part is inconsistent with that of the heart of the to-be-measured object, an accurate measurement result can be obtained. Therefore, this embodiment can greatly improve usability of a blood pressure measurement device, thereby greatly improving user experience. Moreover, for an electronic device with relatively high space flexibility for a measured part, such as a mobile phone or a watch, after this embodiment is used, because a requirement that the height of the measured part is kept consistent with that of the heart of the user no longer needs to be satisfied, improvement in usability is particularly evident.

In an optional implementation, before the second coordinate is determined by using the first coordinate and the first attitude angle in step S102, the first coordinate and the first attitude angle may be first determined.

The first attitude angle represents an attitude angle of the second coordinate system relative to the first coordinate system, as described above. In this embodiment of this application, the first attitude angle may include a pitch angle, a roll angle, and a yaw angle, or may include only a pitch angle and a roll angle. When the first attitude angle is determined, in an optional implementation, the first attitude angle may be determined by using a specific software interface, and the electronic device reads the first attitude angle by using the software interface. In another optional implementation, data of a micro-electro mechanical system (micro-electro mechanical system, MEMS) sensor, a magnetometer, and the like in the electronic device may be collected, and the collected data is calculated, to obtain the attitude angle of the second coordinate system relative to the first coordinate system.

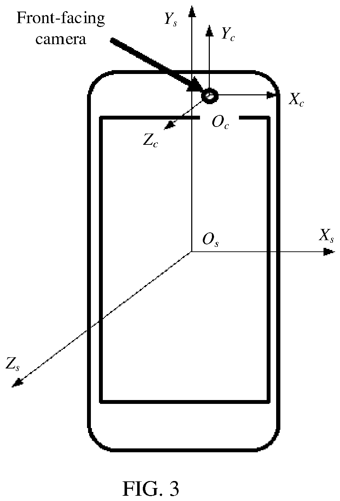

The first coordinate of the facial feature point in the second coordinate system may be determined based on a coordinate of the facial feature point in a fourth coordinate system of the electronic device. Optionally, a lens optical center of a camera that collects a facial image in the electronic device may be used as an origin of the fourth coordinate system, three coordinate axes of the fourth coordinate system are respectively in a same direction as the three coordinate axes of the second coordinate system, and there is a preset translation amount between the fourth coordinate system and the second coordinate system. FIG. 3 is a schematic diagram of the fourth coordinate system and the second coordinate system. As shown in FIG. 3, the monocular front-facing camera of the mobile phone is configured to collect the facial image, $O_sX_sY_sZ_s$ represents a coordinate system of the mobile phone, and $O_cX_cY_cZ_c$ represents the fourth coordinate system of the front-facing camera. X-axes, Y-axes, and Z-axes of the two coordinate systems are respectively in a same direction, and the origin $O_c$ of the fourth coordinate system is located at the lens optical center of the front-facing camera. It can be learned from FIG. 3 that the fourth coordinate system is in a translation relationship with the coordinate system of the mobile phone, and a translation amount between the fourth coordinate system and the coordinate system of the mobile phone may be determined based on a structural size of the mobile phone.

The following describes, by using an example in which the facial feature point is a middle point between pupil centers of two eyes, a process of determining the first coordinate of the facial feature point in the second coordinate system based on the coordinate of the facial feature point in the fourth coordinate system of the electronic device.

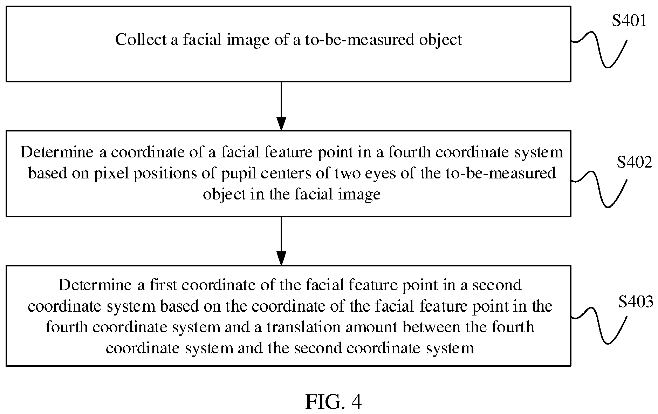

FIG. 4 is a schematic flowchart of a blood pressure processing method according to an embodiment of this application. As shown in FIG. 4, when the facial feature point is the middle point between the pupil centers of the two eyes, a process of determining the first coordinate includes:
S401: Collect the facial image of the to-be-measured object.

Optionally, when the camera of the electronic device collects the facial image of the to-be-measured object, a complete facial image may be collected, or a partial facial image including the facial feature point may be collected.

S402: Determine the coordinate of the facial feature point in the fourth coordinate system based on pixel positions of the pupil centers of the two eyes of the to-be-measured object in the facial image.

Optionally, after the facial image of the to-be-measured object is collected, pixel coordinates of the pupil centers of the two eyes in the facial image may be recognized by using an image recognition method. Based on a point distance between pixels in the facial image, a camera focal length, a pupil distance of the two eyes, and the triangle similarity theorem, the coordinate of the facial feature point in the fourth coordinate system can be further determined.

S403: Determine the first coordinate of the facial feature point in the second coordinate system based on the coordinate of the facial feature point in the fourth coordinate system and the translation amount between the fourth coordinate system and the second coordinate system.

The following further explains the foregoing processing process with reference to a figure.

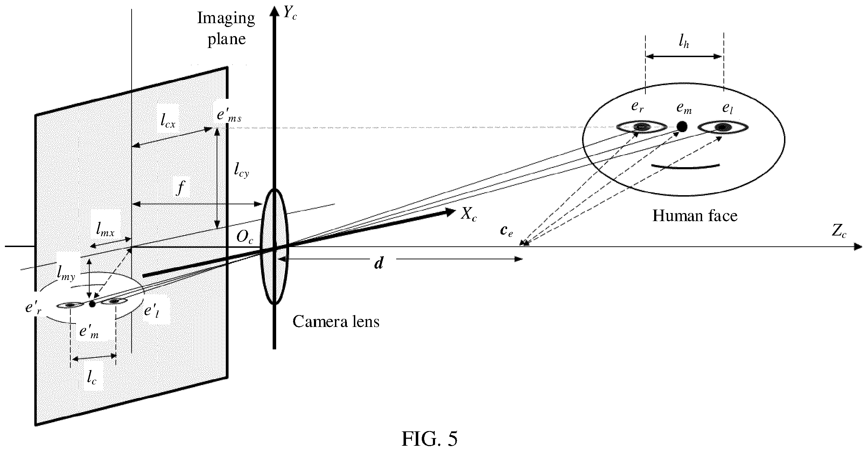

FIG. 5 is a schematic diagram of imaging of a human face in the fourth coordinate system. As shown in FIG. 5, $O_cX_cY_cZ_c$ is the fourth coordinate system, $f$ is the camera focal length, $e_l$, $e_r$, and $e_m$ are respectively the pupil center of the left eye, the pupil center of the right eye, and the middle point between the pupil centers of the two eyes, and $l_h$ is the pupil distance of the two eyes. Assuming that a line connecting the pupil centers of the two eyes is approximately parallel to an imaging plane, $c_e$ represents a perpendicular foot of the pupil center of the left eye, the pupil center of the right eye, and the middle point between the pupil centers of the two eyes on an axis $Z_c$, and d is a distance between the perpendicular foot $c_e$ and the origin $O_c$ of the fourth coordinate system. In the camera imaging plane, $e'_l$, $e'_r$, and $e'_m$ are respectively imaging points of the pupil center of the left eye, the pupil center of the right eye, and the middle point between the pupil centers of the two eyes, $l_c$ is a distance between the imaging points $e'_l$ and $e'_r$ of the pupil centers of the two eyes in the imaging plane, $l_{mx}$ and $l_{my}$ are respectively absolute values of coordinate values $x_f$ and $y_f$ of the imaging point $e'_m$ of the middle point between the pupil centers of the two eyes in the fourth coordinate system, a point $e'_{ms}$ is a vertical projection point of the middle point between the pupil centers of the two eyes in the imaging plane, and $l_{cx}$ and $l_{cy}$ are absolute values of coordinate values $x_c$ and $y_c$ of the point $e'_{ms}$ in the fourth coordinate system. Based on FIG. 5, a plurality of similar triangles may be determined. Based on the triangle similarity theorem, a coordinate value of the facial feature point in the fourth coordinate system may be calculated. Specifically, based on the pupil distance $l_h$ of the two eyes, the distance $l_c$ between the imaging points of the pupil centers of the two eyes in the imaging plane, and the focal length $f$, and based on a side length formula of similar triangles, d may be first calculated. Calculated $d$ is a coordinate value $Z_c$ of the middle point between the pupil centers of the two eyes in the fourth coordinate system, and then $x_c$ and $y_c$ are calculated based on the absolute values $l_{mx}$ and $l_{my}$ that are in the imaging plane and that are of the coordinate values $x_f$ and $y_f$ of the imaging point $e'_m$ of the middle point between the pupil centers of the two eyes in the fourth coordinate system, and $f$ and $d$, and based on the side length formula of the similar triangles.

Further, in the example shown in FIG. 5, manual measurement may be selected as a manner of obtaining the pupil distance $l_h$ of the two eyes, or an empirical value setting manner or another measurement manner may be adopted. The camera focal length f may be obtained by using a parameter of the camera itself. After the foregoing parameters are obtained, $x_c$ and $y_c$ can be calculated based on the foregoing obtained absolute values $l_{mx}$ and $l_{my}$ that are in the imaging plane and that are of the coordinate values $x_f$ and $y_f$ of the imaging point $e'_m$ of the middle point between the pupil centers of the two eyes in the fourth coordinate system, and f and d, and based on the side length formula of the similar triangles. In this way, the coordinate value $(x_c,y_c,z_c)$ of the middle point $e_m$ between the pupil centers of the two eyes in the fourth coordinate system is obtained.

After the coordinate value $(x_c,y_c,z_c)$ of the middle point $e_m$ between the pupil centers of the two eyes in the fourth coordinate system is obtained, a translation vector $T = [x_{cs},y_{cs},z_{cs}]$ from the fourth coordinate system to the second coordinate system may be determined based on the structural size of the mobile phone, and based on a coordinate transformation relationship of a spatial point between different coordinate systems, the first coordinate $(x_s, y_s, z_s)$ of the facial feature point in the second coordinate system can be obtained through summation based on the coordinate of the facial feature point in the fourth coordinate system and the translation vector.

It should be noted that FIG. 5 is merely a simplified equivalent diagram shown for describing a process of determining the coordinate of the facial feature point in the fourth coordinate system, and is not a schematic diagram of an actual imaging effect of the camera.

In addition to the foregoing example, in a case when the facial feature point is another point, the first coordinate may also be determined by using a method similar to the foregoing processing process. For example, when the facial feature point is the pupil center of the left eye of the to-be-measured object, the coordinate of the pupil center of the left eye in the fourth coordinate system may be first determined by using the foregoing method, and based on the translation amount between the fourth coordinate system and the second coordinate system, the first coordinate of the pupil center of the left eye in the second coordinate system is further calculated.

It should be noted that, when the first coordinate of the facial feature point in the second coordinate system is determined based on the coordinate of the facial feature point in the fourth coordinate system, a method for determining the coordinate of the facial feature point in the fourth coordinate system is described by using the monocular front-facing camera as an example. In a specific implementation process, the coordinate of the facial feature point in the fourth coordinate system may alternatively be determined based on a multi-view camera, and the camera may use a ToF technology, a structured light technology, an RGB binocular technology, and the like. In addition, the camera in this embodiment of this application may be a front-facing camera, or may be rear-facing camera. A position of the camera on the electronic device is not limited in this embodiment of this application.

Some optional implementations of determining the first coordinate and the first attitude angle are described above. The second coordinate of the facial feature point in the first coordinate system may be determined based on the first coordinate and the first attitude angle, and the third height difference is determined based on the second height difference and the first height difference that is identified by the second coordinate.

As described above, the second height difference represents the height difference between the facial feature point and the heart of the to-be-measured object. The second height difference may be first determined before the third height difference is determined based on the second height difference and the second coordinate.

In an optional implementation, there is a preset correspondence between the second height difference and at least one parameter.

The at least one parameter is a known parameter, and may be obtained in advance in a manner such as user input, or automatic measurement by the electronic device. The at least one parameter may include the gender, height, weight, and the like of the to-be-measured object.

For example, second height difference information under different genders and heights may be obtained in advance through statistics collection with reference to a large quantity of samples, and processing such as averaging is performed on the statistical information, to obtain information about correspondences between genders and different heights and the second height difference. Further, in this embodiment of this application, before blood pressure measurement is performed, the user may be reminded to first enter gender and/or height information of the to-be-measured object, and further, the second height difference is obtained based on the correspondences between genders/heights and the second height difference.

In another optional implementation, the electronic device may further remind the user to measure the second height difference of the to-be-measured object, and enter the measured second height difference. The electronic device may directly use the second height difference. In this implementation, before performing blood pressure measurement, the electronic device may remind the user to manually enter the second height difference. In addition, the user may further enter another parameter, for example, the pupil distance of the two eyes of the to-be-measured object, for use in the foregoing processing process in this embodiment of this application.

In addition to the foregoing two optional implementations, the second height difference may alternatively be a default value. The electronic device pre-stores the default value. In this embodiment of this application, the second height difference is directly obtained, and the third height difference is determined based on the second height difference.

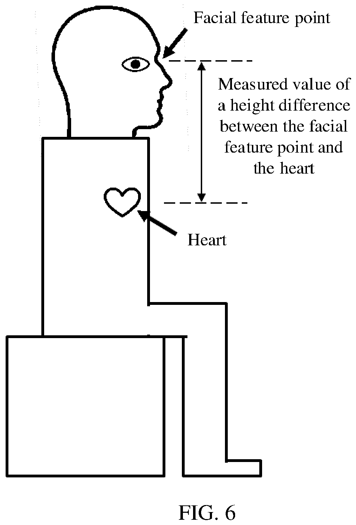

The foregoing optional implementations may be used for a case in which measurement is performed on the to-be-measured object in a posture in which the torso and the head are perpendicular to a ground. FIG. 6 is a schematic diagram of a posture in which the torso and the head are perpendicular to the ground. In the posture shown in FIG. 6, a relatively accurate second height difference may be obtained in the foregoing optional implementations.

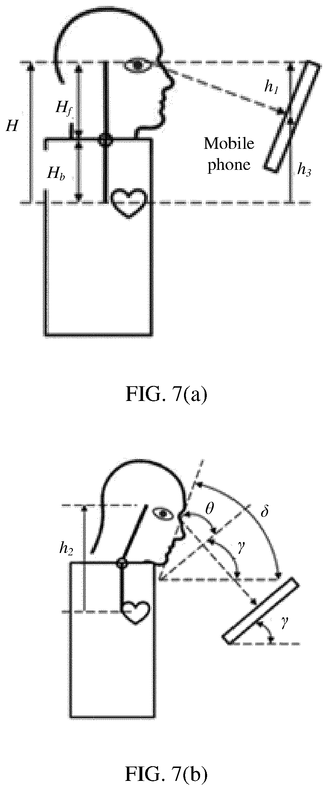

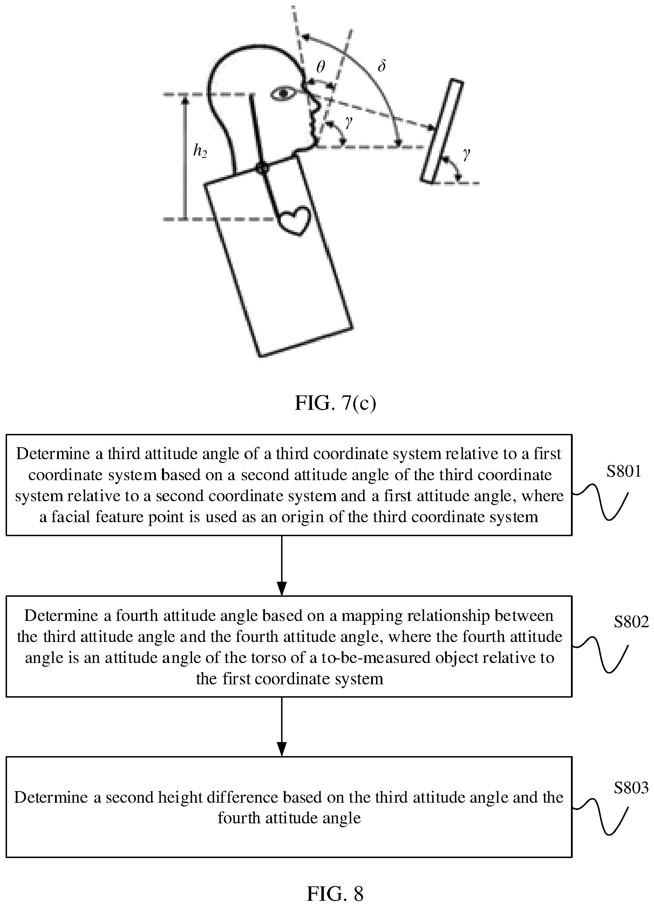

In a specific implementation process, the posture of the to-be-measured object may be the posture in which the torso and the head are perpendicular to the ground shown in FIG. 6, or may be a posture in which the torso or the head is not perpendicular to the ground, for example, the torso is upright and the head is tilted downward, or the torso leans backward. FIG. 7(a), FIG. 7(b), and FIG. 7(c) are respectively schematic diagrams of three measurement postures. FIG. 7(a) is a schematic diagram of a posture in which a torso and a head are perpendicular to a ground, namely, the posture shown in FIG. 6. FIG. 7(b) is a schematic diagram of a posture in which the torso is upright and the head is tilted downward. FIG. 7(c) is a schematic diagram of a posture in which the torso leans backward.

There is an error between the second height difference when the posture of the to-be-measured object is the posture in which the torso or the head is not perpendicular to the ground and the second height difference when a standard measurement posture is present, and the error of the second height difference can be greatly reduced in the following optional implementations, so that an accurate second height difference can be obtained in various postures of the to-be-measured object.

FIG. 8 is a schematic flowchart of a blood pressure processing method according to an embodiment of this application. As shown in FIG. 8, another implementation of determining the second height difference includes the following steps.

S801: Determine a third attitude angle of a third coordinate system relative to the first coordinate system based on a second attitude angle of the third coordinate system relative to the second coordinate system and the first attitude angle, where the facial feature point is used as an origin of the third coordinate system.

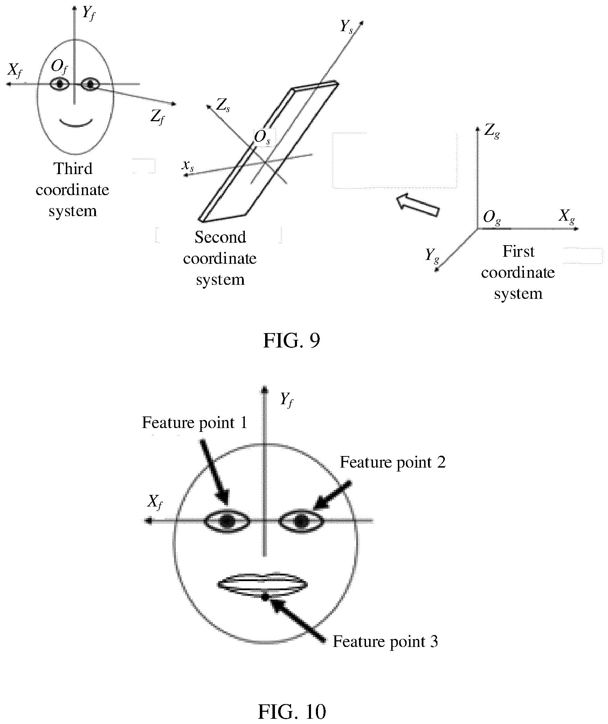

FIG. 9 is a schematic diagram of estimating the third attitude angle. It should be noted that, to clearly distinguish the first coordinate system from the second coordinate system, the origin of the first coordinate system and the origin of the second coordinate system shown in FIG. 9 are not located at a same position, but actually the origins overlap. As shown in FIG. 9, $O_f X_f Y_f Z_f$ is the third coordinate system, the origin $O_f$ of the third coordinate system is located at the middle point between the pupil centers of the two eyes, an axis $X_f$ points to a direction from the origin $O_f$ to the right eye, an axis $Y_f$ points to a direction upward relative to the head, and an axis $Z_f$ points to a direction that is perpendicular to a plane $O_f X_f Y_f$ and outward. $O_s X_s Y_s Z_s$ is the second coordinate system, and $O_g X_g Y_g Z_g$ is the first coordinate system. First, the first attitude angle, namely, the attitude angle of the electronic device relative to the first coordinate system, may be obtained in the foregoing manner. Second, the second attitude angle of the third coordinate system relative to the second coordinate system may be determined. Optionally, a plurality of facial feature points of the to-be-measured object may be selected, and the third coordinate system is determined based on the plurality of facial feature points. In addition, an attitude angle of the third coordinate system relative to the fourth coordinate system may be obtained based on a coordinate value of each of the plurality of selected facial feature points in the fourth coordinate system. Because the coordinate axes of the fourth coordinate system are respectively in a same direction as the coordinate axes of the second coordinate system, the attitude angle of the third coordinate axis relative to the fourth coordinate axis is the second attitude angle of the third coordinate system relative to the second coordinate system.

Optionally, when the plurality of facial feature points of the to-be-measured object are selected, a plurality of feature points that are not on a same straight line may be selected, and a quantity of the selected feature points may be, for example, 3 or a value greater than 3. FIG. 10 is an example of selecting a plurality of facial feature points. As shown in FIG. 10, three feature points are selected, where the pupil center of the right eye is selected as a feature point 1, the pupil center of the left eye is selected as a feature point 2, and a central position (a position shown by a black dot in the figure) of a lip line of the lower lip is selected as a feature point 3. The third coordinate system is determined based on the three feature points, and the second attitude angle of the third coordinate system relative to the second coordinate system is further obtained based on coordinate values of the three feature points in the fourth coordinate system.

On the basis of obtaining the second attitude angle and the first attitude angle, optionally, the third attitude angle of the third coordinate system relative to the first coordinate system may be determined through rotation transformation.

S802: Determine a fourth attitude angle based on a mapping relationship between the third attitude angle and the fourth attitude angle, where the fourth attitude angle is an attitude angle of the torso of the to-be-measured object relative to the first coordinate system.

For example, the to-be-measured object is a person. In different postures of the person, probability distributions of the third attitude angle and the fourth attitude angle satisfy correspondences of probability statistical distributions. For example, during blood pressure measurement by using a mobile phone, a person is usually used to sitting upright and holding the mobile phone at a position below the head, and at the same time, the human face is tilted downward by a particular angle to view the screen or is approximately facing forward. This posture corresponds to the posture shown in FIG. 7(b). Therefore, when it is detected that the human face is tilted downward by a particular angle or is approximately facing forward during blood pressure measurement, it may be considered that the torso of the person is in an upright-sitting or upright-standing posture with a relatively high probability. In another example, when it is detected that the human face is tilted upward, it may be considered that the torso leans backward, and this posture corresponds to the posture shown in FIG. 7(c). In the foregoing example, the tilting angle of the human face may be represented by the third attitude angle, and a tilting degree of the torso may be represented by the fourth attitude angle.

Based on the foregoing correspondence, the mapping relationship between the third attitude angle and the fourth attitude angle may be constructed. Optionally, the mapping relationship between the third attitude angle and the fourth attitude angle may be constructed by using a mathematical model of a probability distribution function or a mapping relationship table of the probability distribution function. Table 1 below is an example of a mapping relationship table between the third attitude angle and a probability distribution function of the fourth attitude angle. As shown in Table 1, a specific third attitude angle corresponds to a probability distribution function using the third attitude angle as a parameter, and a fourth attitude angle may be obtained based on the function.

**Table 1**

| Third attitude angle | Probability distribution function of a fourth attitude angle |
|---|---|
| $[\theta_f^1, \psi_f^1, \phi_f^1]$ | $f_1(\theta_b^1, \psi_b^1, \phi_b^1)$ |
| ... | ... |

(continued)

| Third attitude angle | Probability distribution function of a fourth attitude angle |
|---|---|
| $\left[\theta_f^N, \psi_f^N, \phi_f^N\right]$ | $f_1\left(\theta_b^N, \psi_b^N, \phi_b^N\right)$ |

S803: Determine the second height difference based on the third attitude angle and the fourth attitude angle.

In an optional implementation, the second height difference may be determined based on the third attitude angle, the fourth attitude angle, a preset height difference, and preset ratio information. The preset height difference is a height difference between the facial feature point and the heart of the to-be-measured object when the torso and the head of the to-be-measured object are vertical, and the preset ratio information is a ratio of a height difference between the facial feature point and a neck preset point of the to-be-measured object to the preset height difference when the torso and the head of the to-be-measured object are vertical.

For example, the neck preset point may be a rotation center of the neck relative to the torso when the head of the to-be-measured object moves.

Still refer to FIG. 7(a). H is the foregoing preset height difference, $H_f$ is the height difference between the facial feature point and the neck preset point, and $H_b$ is a height difference between the neck preset point and the heart. Assuming that the foregoing preset ratio is $r_f$, in this embodiment, the second height difference may be determined based on a trigonometric function relationship. For example, the height difference $H_f$ between the facial feature point and the neck preset point and the height difference $H_b$ between the neck preset point and the heart when the human face and the torso are in a vertical state may be calculated based on H and $r_f$. First, an actual height difference $h_f$ between the facial feature point and the preset neck point during blood pressure measurement is calculated by using the triangular relationship based on $H_f$ and the third attitude angle, and then, based on the third attitude angle, the probability distribution function that is of the fourth attitude angle and that corresponds to the third attitude angle is determined by using the method in the foregoing embodiment. When the fourth attitude angle is a specific value, an actual height difference $h_b$ between the neck preset point and the heart during blood pressure measurement may be determined with reference to the height difference $H_b$ between the neck preset point and the heart. After the probability distribution of the fourth attitude angle is determined, a probability distribution function of the actual height difference $h_b$ between the neck preset point and the heart during blood pressure measurement may be calculated. Further, it can be learned from FIG. 7(a) that a sum of $h_b$ and $h_f$ is the height difference $h_2$ between the facial feature point and the heart during blood pressure measurement, namely, the second height difference. Therefore, a probability distribution function of the height difference $h_2$ between the facial feature point and the heart during blood pressure measurement can be determined. Based on the function, a maximum value of a likelihood probability of the height difference between the facial feature point and the heart during blood pressure measurement may be calculated, and the value is used as the second height difference $h_2$, or an expected value of the height difference between the facial feature point and the heart during blood pressure measurement may alternatively be calculated, and the value is used as the second height difference $h_2$.

In another optional implementation, the second height difference may alternatively be determined based on the third attitude angle, the fourth attitude angle, the height difference between the facial feature point and the neck preset point, and the height difference between the neck preset point and the heart.

Some optional implementations of determining the second height difference are described above. The third height difference may be determined based on the second height difference and the foregoing second coordinate.

It can be learned from the foregoing that the third height difference is the height difference between the origin of the second coordinate system and the heart of the to-be-measured object, and the third height difference affects accuracy of the blood pressure measurement result. Therefore, the blood pressure measurement result obtained based on the signal collected by the blood pressure measurement signal collection apparatus may be compensated based on the third height difference, to obtain an accurate blood pressure measurement result.

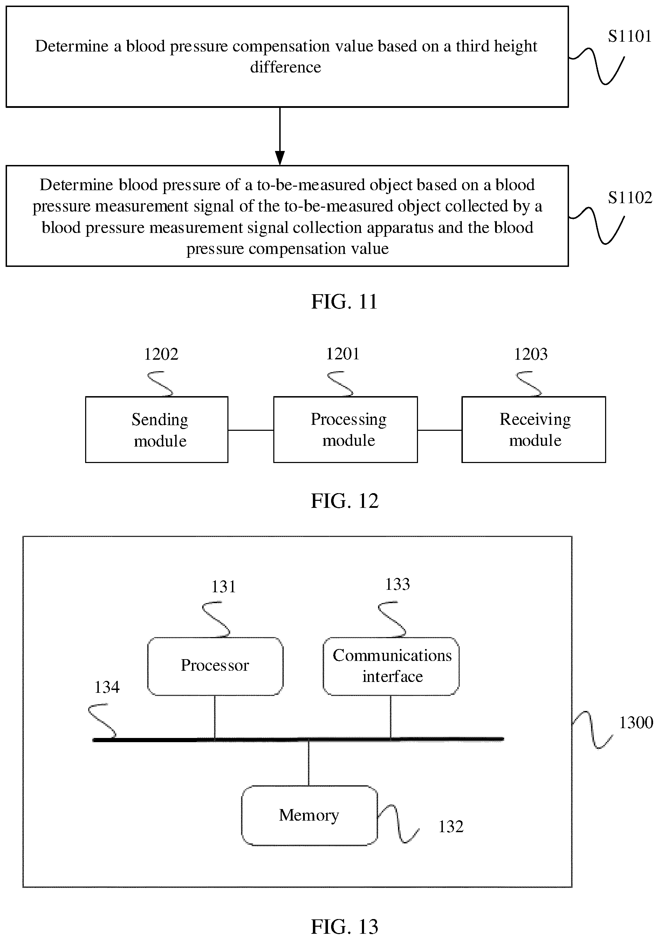

FIG. 11 is a schematic flowchart of a blood pressure processing method according to an embodiment of this application. As shown in FIG. 11, a process of determining the blood pressure of the to-be-measured object based on the blood pressure measurement signal of the to-be-measured object collected by the blood pressure measurement signal collection apparatus and the third height difference includes the following steps.

S1101: Determine a blood pressure compensation value based on the third height difference.

Optionally, the blood pressure compensation value may be determined based on the third height difference and a preset blood pressure compensation model. The blood pressure compensation model is used for representing a correspondence between the third height difference and the blood pressure compensation value.

In an example, the blood pressure compensation model may be established based on an internal relationship in which a height change of the electronic device relative to the heart causes a change to vascular blood pressure of the part on which blood pressure measurement is performed.

In another example, the blood pressure compensation model may be established based on actual measurement data. Specifically, blood pressure error information under a plurality of third height differences is first collected, and the blood pressure compensation model is further established based on the blood pressure error information under the plurality of third height differences. Optionally, a method for establishing the blood pressure compensation model may include: calculating an average value of blood pressure error data corresponding to each of the third height differences, or calculating a probability density function of the blood pressure error data corresponding to each of the third height differences, calculating an expected error value based on the probability density function, and performing function fitting for all the third height differences and corresponding expected error values of blood pressure. The function obtained through fitting is the blood pressure compensation model.

S1102: Determine the blood pressure of the to-be-measured object based on the blood pressure measurement signal of the to-be-measured object collected by the blood pressure measurement signal collection apparatus and the blood pressure compensation value.

After the blood pressure measurement signal collection apparatus collects the blood pressure measurement signal, the electronic device may obtain a blood pressure measurement value by processing the signal. On this basis, accurate blood pressure of the to-be-measured object may be obtained by superimposing the blood pressure measurement value and the blood pressure compensation value.

It should be noted that the blood pressure compensation value may be a positive value or a negative value.

In a specific implementation process, in a process of measuring blood pressure of the to-be-measured object by using the method in this embodiment of this application, a posture of the to-be-measured object should be kept consistent. To be specific, after the third height difference is calculated by using the method in this embodiment of this application, the third height difference should be kept unchanged, to ensure an accurate compensation result. Correspondingly, in the process of measuring the blood pressure of the to-be-measured object, the electronic device may detect, based on a specific periodicity, whether a height of the electronic device changes when the blood pressure measurement signal collection apparatus collects the blood pressure measurement signal, and if the height of the electronic device changes when the blood pressure measurement signal collection apparatus collects the blood pressure measurement signal, the blood pressure measurement signal collection apparatus stops collecting the blood pressure measurement signal. At the same time, the user is prompted that measurement is stopped and the user should keep the same posture during next measurement. Optionally, the electronic device may detect, by using a motion sensor, a barometer, or the like, whether the foregoing height changes.

FIG. 12 is a schematic structural diagram of an apparatus for processing blood pressure measurement according to an embodiment of this application. As shown in FIG. 12, the apparatus includes a processing module 1201.

The processing module is configured to: obtain a facial feature point of a to-be-measured object;

determine a first height difference between the facial feature point and an origin of a second coordinate system based on a first coordinate of the facial feature point in the second coordinate system and a first attitude angle of the second coordinate system relative to a first coordinate system, where a preset point on an electronic device is used as the origin of the second coordinate system;

determine a third height difference between the origin of the second coordinate system and the heart of the object based on the first height difference and a second height difference, where the second height difference is a height difference between the facial feature point and the heart of the object; and

determine blood pressure of the object based on a blood pressure measurement signal of the object collected by a blood pressure measurement signal collection apparatus and the third height difference.

Optionally, the apparatus further includes a sending module 1202 and a receiving module 1203. The sending module 1202 is configured to send information to an external device, and the receiving module 1203 is configured to receive information sent by the external device or entered by a user.

In a possible implementation, the processing module 1201 is further configured to:
determine the first coordinate of the facial feature point in the second coordinate system based on a coordinate of the facial feature point in a fourth coordinate system of the electronic device, where a coordinate axis of the fourth coordinate system is collinear with an optical axis, and there is a preset translation amount between the fourth coordinate system and the second coordinate system.

In a possible implementation, when the facial feature point is the middle point between pupil centers of two eyes of the object, the processing module 1201 may be specifically configured to:

collect a facial image of the to-be-measured object;
determine the coordinate of the facial feature point in the fourth coordinate system based on pixel positions of the pupil centers of the two eyes of the to-be-measured object in the facial image; and

determine the first coordinate of the facial feature point in the second coordinate system based on the coordinate of the facial feature point in the fourth coordinate system and the translation amount between the fourth coordinate system and the second coordinate system.

In a possible implementation, the processing module 1201 is further configured to:
determine the second height difference.
In a possible implementation, there is a preset correspondence between the second height difference and at least one parameter.
In a possible implementation, the processing module 1201 is specifically configured to:
receive configuration parameter information entered by the object, where the configuration parameter information includes the second height difference.
In a possible implementation, the processing module 1201 is specifically configured to:

determine a third attitude angle of a third coordinate system relative to the first coordinate system based on a second attitude angle of the third coordinate system relative to the second coordinate system and the first attitude angle, where the facial feature point is used as an origin of the third coordinate system;
determine a fourth attitude angle based on a mapping relationship between the third attitude angle and the fourth attitude angle, where the fourth attitude angle is an attitude angle of the torso of the to-be-measured object relative to the first coordinate system; and
determine the second height difference based on the third attitude angle and the fourth attitude angle.

In a possible implementation, the processing module 1201 is specifically configured to:

determine the second height difference based on the third attitude angle, the fourth attitude angle, a preset height difference, and preset ratio information, where
the preset height difference is a height difference between the facial feature point and the heart of the object when the torso and the head of the object are vertical, and the preset ratio information is a ratio of a height difference between the facial feature point and a neck preset point of the object to the preset height difference when the torso and the head of the object are vertical.

In a possible implementation, the processing module 1201 is specifically configured to:
determine the second height difference based on the third attitude angle, the fourth attitude angle, a height difference between the facial feature point and a neck preset point, and a height difference between the neck preset point and the heart.
In a possible implementation, the processing module 1201 is specifically configured to:

determine a blood pressure compensation value based on the third height difference; and
determine the blood pressure of the object based on the blood pressure measurement signal of the object collected by the blood pressure measurement signal collection apparatus and the blood pressure compensation value.

In a possible implementation, the processing module 1201 is specifically configured to:
determine the blood pressure compensation value based on the third height difference and a preset blood pressure compensation model, where the blood pressure compensation model is used for representing a correspondence between the third height difference and the blood pressure compensation value.
In a possible implementation, the processing module 1201 is further configured to:

collect blood pressure error information under a plurality of third height differences; and
establish the blood pressure compensation model based on the blood pressure error information under the plurality of third height differences.

In a possible implementation, the processing module 1201 is further configured to:
detect whether a height of the electronic device changes when the blood pressure measurement signal collection apparatus collects the blood pressure measurement signal, and stop collecting the blood pressure measurement signal when the height of the electronic device changes.
The apparatus for processing blood pressure measurement provided in this embodiment of this application may execute actions of the electronic device in the foregoing method embodiment. Implementation principles and technical effects of the apparatus for processing blood pressure measurement are similar to those of the electronic device, and are not described herein again.

It should be noted that, it should be understood that division of the modules of the foregoing apparatus is merely division of logical functions, and in actual implementation, all or some modules may be integrated into one physical entity, or may be physically separated. In addition, all of these modules may be implemented in a form of invoking software by processing elements; or all of these modules may be implemented in a form of hardware; or some modules are implemented in a form of invoking software by processing elements, and some modules are implemented in a form of hardware. For example, the processing module may be a separately disposed processing element, or may be integrated into a chip of the foregoing apparatus for implementation. In addition, the processing module may alternatively be stored in a memory of the foregoing apparatus in a form of program code, and a processing element of the foregoing apparatus invokes and executes a function of the foregoing determining module. Implementation of other modules is similar to that of the processing module. In addition, all or some of these modules may be integrated together, or may be implemented separately. The processing element described herein may be an integrated circuit having a signal processing capability. In an implementation process, the steps of the foregoing method or the foregoing modules may be completed by using an integrated logic circuit of hardware in the processing elements or instructions in a form of software.

For example, the foregoing modules may be one or more integrated circuits configured to implement the foregoing method, for example, one or more application-specific integrated circuits (application-specific integrated circuits, ASICs), or one or more microprocessors (digital signal processors, DSPs), or one or more field programmable gate arrays (field programmable gate arrays, FPGAs), or the like. In another example, when one of the foregoing modules is implemented in a form of invoking program code by a processing element, the processing element may be a general-purpose processor, for example, a central processing unit (central processing unit, CPU) or another processor that can invoke the program code. In another example, these modules may be integrated together and implemented in a form of a system-on-$\alpha$-chip (system-on-$\alpha$-chip, SOC).

In the foregoing embodiment, the modules may be entirely or partially implemented by using software, hardware, firmware, or any combination thereof. When software is used for implementation, the modules may be entirely or partially implemented in a form of a computer program product. The computer program product includes one or more computer instructions. When the computer program instructions are loaded and executed on a computer, procedures or functions according to the embodiments of this application are entirely or partially generated. The computer may be a general-purpose computer, a special-purpose computer, a computer network, or another programmable apparatus. The computer instructions may be stored in a computer-readable storage medium, or transmitted from one computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, wireless, or microwaves) manner. The computer-readable storage medium may be any usable medium that can be accessed by the computer, or a data storage device such as a server or a data center that integrates one or more usable media. The usable medium may be a magnetic medium (for example, a floppy disk, a hard disk, or a magnetic tape), an optical medium (for example, a DVD), a semiconductor medium (for example, a solid state disk (SSD)), or the like.

FIG. 13 is a schematic structural diagram of an electronic device 1300 according to an embodiment of this application. As shown in FIG. 13, the electronic device may include a processor 131, a memory 132, a communications interface 133, and a system bus 134. The memory 132 and the communications interface 133 are connected to the processor 131 by using the system bus 134 and complete mutual communication. The memory 132 is configured to store computer-executable instructions. The communications interface 133 is configured to communicate with another device. When the processor 131 executes the computer programs, the solutions of the embodiment shown in FIG. 1 to FIG. 11 are implemented.

The system bus mentioned in FIG. 13 may be a peripheral component interconnect (peripheral component interconnect, PCI) bus or an extended industry standard architecture (extended industry standard architecture, EISA) bus, or the like. The system bus may be divided into an address bus, a data bus, a control bus, or the like. For ease of representation, in the figure, only one thick line is used to represent the system bus, but it does not mean that there is only one bus or one type of bus. The communications interface is configured to implement communication between a database access apparatus and another device (for example, a client, a read/write library, or a read-only library). The memory may include a random access memory (random access memory, RAM), or may further include a non-volatile memory (non-volatile memory), for example, at least one disk memory.

The processor may be a general-purpose processor, including a central processing unit (CPU), a network processor (network processor, NP), and the like. The processor may alternatively be a digital signal processor (DSP), an application-specific integrated circuit (ASIC), a field programmable gate array (FPGA) or another programmable logic device, a discrete gate or a transistor logic device, or a discrete hardware component.

Optionally, an embodiment of this application further provides a storage medium. The storage medium stores instructions. When the instructions run on a computer, the computer is enabled to perform the method in the embodiment shown in FIG. 1 to FIG. 11.

Optionally, an embodiment of this application further provides a chip for running an instruction. The chip is configured to perform the method in the embodiment shown in FIG. 2 to FIG. 5.

An embodiment of this application further provides a program product. The program product includes computer programs. The computer programs are stored in a storage medium. At least one processor may read the computer programs from the storage medium. When the at least one processor executes the computer programs, the method in the embodiment shown in FIG. 1 to FIG. 11 may be implemented.

In the embodiments of this application, "at least one" means one or more, and "a plurality of" means two or more. "And/or" describes an association relationship between associated objects, and represents that three relationships may exist. For example, A and/or B may represent: Only A exists, both A and B exist, and only B exists, where A and B may be singular or plural. The character "/" generally indicates that the associated objects are in an "or" relationship. In a formula, the character "/" indicates that the associated objects are in a "division" relationship. "At least one of the following items" or its similar expressions mean any combination of these items, including any combination of a single item or a plurality of items. For example, at least one of a, b, and c may represent a, b, c, a-b, a-c, b-c, or a-b-c, where a, b, and c may be singular or plural.

It may be understood that various numeric numbers in the embodiments of this application are merely distinguished for ease of description, and are not intended to limit the scope of the embodiments of this application.

It may be understood that, in the embodiments of this application, sequence numbers of the foregoing processes do not mean execution sequences. The execution sequences of the processes should be determined based on functions and internal logic of the processes, and should not constitute any limitation on the implementation processes of the embodiments of this application.

**Claims**

1. A method for processing blood pressure measurement, comprising:

   obtaining a facial feature point of a to-be-measured object;
   determining a first height difference between the facial feature point and an origin of a second coordinate system based on a first coordinate of the facial feature point in the second coordinate system and a first attitude angle of the second coordinate system relative to a first coordinate system, wherein a preset point on an electronic device is used as the origin of the second coordinate system;
   determining a third height difference between the origin of the second coordinate system and the heart of the object based on the first height difference and a second height difference, wherein the second height difference is a height difference between the facial feature point and the heart of the object; and
   determining blood pressure of the object based on a blood pressure measurement signal of the object collected by a blood pressure measurement signal collection apparatus and the third height difference.

2. The method according to claim 1, before the determining a first height difference between the facial feature point and an origin of a second coordinate system based on a first coordinate of the facial feature point in the second coordinate system and a first attitude angle of the second coordinate system relative to a first coordinate system, further comprising:
   determining the first coordinate of the facial feature point in the second coordinate system based on a coordinate of the facial feature point in a fourth coordinate system of the electronic device, wherein a coordinate axis of the fourth coordinate system is collinear with an optical axis, and there is a preset translation amount between the fourth coordinate system and the second coordinate system.

3. The method according to claim 2, wherein the facial feature point is a middle point between pupil centers of two eyes of the object; and
   the determining the first coordinate of the facial feature point in the second coordinate system based on a coordinate of the facial feature point in a fourth coordinate system of the electronic device comprises:

   collecting a facial image of the to-be-measured object;
   determining the coordinate of the facial feature point in the fourth coordinate system based on pixel positions of the pupil centers of the two eyes of the to-be-measured object in the facial image; and
   determining the first coordinate of the facial feature point in the second coordinate system based on the coordinate of the facial feature point in the fourth coordinate system and the translation amount between the fourth coordinate system and the second coordinate system.

4. The method according to any one of claims 1 to 3, before the determining a third height difference between the origin of the second coordinate system and the heart of the object based on the second coordinate and a second height difference, further comprising:

determining the second height difference.

5. The method according to claim 4, wherein there is a preset correspondence between the second height difference and at least one parameter.

6. The method according to claim 4, wherein the determining the second height difference comprises:
receiving configuration parameter information entered by the object, wherein the configuration parameter information comprises the second height difference.

7. The method according to claim 4, wherein the determining the second height difference comprises:

determining a third attitude angle of a third coordinate system relative to the first coordinate system based on a second attitude angle of the third coordinate system relative to the second coordinate system and the first attitude angle, wherein the facial feature point is used as an origin of the third coordinate system;
determining a fourth attitude angle based on a mapping relationship between the third attitude angle and the fourth attitude angle, wherein the fourth attitude angle is an attitude angle of the torso of the to-be-measured object relative to the first coordinate system; and
determining the second height difference based on the third attitude angle and the fourth attitude angle.

8. The method according to claim 7, wherein the determining the second height difference based on the third attitude angle and the fourth attitude angle comprises:

determining the second height difference based on the third attitude angle, the fourth attitude angle, a preset height difference, and preset ratio information, wherein
the preset height difference is a height difference between the facial feature point and the heart of the object when the torso and the head of the object are vertical, and the preset ratio information is a ratio of a height difference between the facial feature point and a neck preset point of the object to the preset height difference when the torso and the head of the object are vertical.

9. The method according to claim 7, wherein the determining the second height difference based on the third attitude angle and the fourth attitude angle comprises:
determining the second height difference based on the third attitude angle, the fourth attitude angle, a height difference between the facial feature point and a neck preset point, and a height difference between the neck preset point and the heart.

10. The method according to any one of claims 1 to 9, wherein the determining blood pressure of the object based on a blood pressure measurement signal of the object collected by a blood pressure measurement signal collection apparatus and the third height difference comprises:

determining a blood pressure compensation value based on the third height difference; and
determining the blood pressure of the object based on the blood pressure measurement signal of the object collected by the blood pressure measurement signal collection apparatus and the blood pressure compensation value.

11. The method according to claim 10, wherein the determining a blood pressure compensation value based on the third height difference comprises:
determining the blood pressure compensation value based on the third height difference and a preset blood pressure compensation model, wherein the blood pressure compensation model is used for representing a correspondence between the third height difference and the blood pressure compensation value.

12. The method according to claim 11, before the determining the blood pressure compensation value based on the third height difference and a preset blood pressure compensation model, further comprising:

collecting blood pressure error information under a plurality of third height differences; and
establishing the blood pressure compensation model based on the blood pressure error information under the plurality of third height differences.

**13.** The method according to any one of claims 1 to 12, further comprising:
detecting whether a height of the electronic device changes when the blood pressure measurement signal collection apparatus collects the blood pressure measurement signal, and stopping collecting the blood pressure measurement signal when the height of the electronic device changes.

**14.** An apparatus for processing blood pressure measurement, comprising: a processing module, wherein

the processing module is configured to: obtain a facial feature point of a to-be-measured object;
determine a first height difference between the facial feature point and an origin of a second coordinate system based on a first coordinate of the facial feature point in the second coordinate system and a first attitude angle of the second coordinate system relative to a first coordinate system, wherein a preset point on an electronic device is used as the origin of the second coordinate system;
determine a third height difference between the origin of the second coordinate system and the heart of the object based on the first height difference and a second height difference, wherein the second height difference is a height difference between the facial feature point and the heart of the object; and
determine blood pressure of the object based on a blood pressure measurement signal of the object collected by a blood pressure measurement signal collection apparatus and the third height difference.

**15.** The apparatus according to claim 14, wherein the processing module is further configured to:
determine the first coordinate of the facial feature point in the second coordinate system based on a coordinate of the facial feature point in a fourth coordinate system of the electronic device, wherein a coordinate axis of the fourth coordinate system is collinear with an optical axis, and there is a preset translation amount between the fourth coordinate system and the second coordinate system.

**16.** The apparatus according to claim 15, wherein the facial feature point is a middle point between pupil centers of two eyes of the object; and
the processing module is specifically configured to:

collect a facial image of the to-be-measured object;
determine the coordinate of the facial feature point in the fourth coordinate system based on pixel positions of the pupil centers of the two eyes of the to-be-measured object in the facial image; and
determine the first coordinate of the facial feature point in the second coordinate system based on the coordinate of the facial feature point in the fourth coordinate system and the translation amount between the fourth coordinate system and the second coordinate system.

**17.** The apparatus according to any one of claims 14 to 16, wherein the processing module is further configured to:
determine the second height difference.

**18.** The apparatus according to claim 17, wherein there is a preset correspondence between the second height difference and at least one parameter.

**19.** The apparatus according to claim 17, wherein the processing module is specifically configured to:
receive configuration parameter information entered by the object, wherein the configuration parameter information comprises the second height difference.

**20.** The apparatus according to claim 17, wherein the processing module is specifically configured to:

determine a third attitude angle of a third coordinate system relative to the first coordinate system based on a second attitude angle of the third coordinate system relative to the second coordinate system and the first attitude angle, wherein the facial feature point is used as an origin of the third coordinate system;
determine a fourth attitude angle based on a mapping relationship between the third attitude angle and the fourth attitude angle, wherein the fourth attitude angle is an attitude angle of the torso of the to-be-measured object relative to the first coordinate system; and
determine the second height difference based on the third attitude angle and the fourth attitude angle.

**21.** The apparatus according to claim 20, wherein the processing module is specifically configured to:

determine the second height difference based on the third attitude angle, the fourth attitude angle, a preset height difference, and preset ratio information, wherein
the preset height difference is a height difference between the facial feature point and the heart of the object when the torso and the head of the object are vertical, and the preset ratio information is a ratio of a height difference between the facial feature point and a neck preset point of the object to the preset height difference when the torso and the head of the object are vertical.

22. The apparatus according to claim 20, wherein the processing module is specifically configured to:
determine the second height difference based on the third attitude angle, the fourth attitude angle, a height difference between the facial feature point and a neck preset point, and a height difference between the neck preset point and the heart.

23. The apparatus according to any one of claims 14 to 22, wherein the processing module is specifically configured to:

    determine a blood pressure compensation value based on the third height difference; and
    determine the blood pressure of the object based on the blood pressure measurement signal of the object collected by the blood pressure measurement signal collection apparatus and the blood pressure compensation value.

24. The apparatus according to claim 23, wherein the processing module is specifically configured to:
determine the blood pressure compensation value based on the third height difference and a preset blood pressure compensation model, wherein the blood pressure compensation model is used for representing a correspondence between the third height difference and the blood pressure compensation value.

25. The apparatus according to claim 24, wherein the processing module is further configured to:

    collect blood pressure error information under a plurality of third height differences; and
    establish the blood pressure compensation model based on the blood pressure error information under the plurality of third height differences.

26. The apparatus according to any one of claims 14 to 25, wherein the processing module is further configured to:
detect whether a height of the electronic device changes when the blood pressure measurement signal collection apparatus collects the blood pressure measurement signal, and stop collecting the blood pressure measurement signal when the height of the electronic device changes.

27. An electronic device, comprising a memory and a processor, wherein
the memory is configured to store computer-executable program code, the program code comprises instructions, and when the processor executes the instructions, the instructions enable the electronic device to perform the method according to any one of claims 1 to 13.

28. A computer program product, wherein the computer program product comprises computer program code, and when the computer program code is executed by a computer, the computer is enabled to perform the method according to any one of claims 1 to 13.

29. A computer-readable storage medium, wherein the computer storage medium stores computer instructions, and when the computer instructions are executed by a computer, the computer is enabled to perform the method according to any one of claims 1 to 19.

Obtain a facial feature point of a to-be-measured object ⟶ S101

Determine a first height difference between the facial feature point and an origin of a second coordinate system based on a first coordinate of the facial feature point in the second coordinate system and a first attitude angle of the second coordinate system relative to a first coordinate system, where a preset point on an electronic device is used as the origin of the second coordinate system ⟶ S102

Determine a third height difference between the origin of the second coordinate system and a heart of the object based on the first height difference and a second height difference, where the second height difference is a height difference between the facial feature point and the heart of the object ⟶ S103

Determine blood pressure of the object based on a blood pressure measurement signal of the object collected by a blood pressure measurement signal collection apparatus and the third height difference ⟶ S104

FIG. 1

$e_m(x_s, y_s, z_s)$

$Y_s$

$Z_s$

$h_1$

$X_s$

Screen side of a
mobile phone

$O_s$

Second
coordinate
system

$h_2$

$h_3$

Heart

$Z_g$

$O_g$

$X_g$

First coordinate
system

$Y_g$

FIG. 2

Front-facing camera

$Y_s$

$Y_c$

$X_c$

$O_c$

$Z_c$

$O_s$

$X_s$

$Z_s$

FIG. 3

| Collect a facial image of a to-be-measured object | S401 |

| Determine a coordinate of a facial feature point in a fourth coordinate system based on pixel positions of pupil centers of two eyes of the to-be-measured object in the facial image | S402 |

| Determine a first coordinate of the facial feature point in a second coordinate system based on the coordinate of the facial feature point in the fourth coordinate system and a translation amount between the fourth coordinate system and the second coordinate system | S403 |

FIG. 4

FIG. 5

Facial feature point

Measured value of
a height difference
between the facial
feature point and
the heart

Heart

FIG. 6

FIG. 7(a)

FIG. 7(b)

FIG. 7(c)

| | |
|---|---|
| Determine a third attitude angle of a third coordinate system relative to a first coordinate system based on a second attitude angle of the third coordinate system relative to a second coordinate system and a first attitude angle, where a facial feature point is used as an origin of the third coordinate system | S801 |
| Determine a fourth attitude angle based on a mapping relationship between the third attitude angle and the fourth attitude angle, where the fourth attitude angle is an attitude angle of the torso of a to-be-measured object relative to the first coordinate system | S802 |
| Determine a second height difference based on the third attitude angle and the fourth attitude angle | S803 |

FIG. 8

FIG. 9

FIG. 10

Determine a blood pressure compensation value based on a third height difference

S1101

Determine blood pressure of a to-be-measured object based on a blood pressure measurement signal of the to-be-measured object collected by a blood pressure measurement signal collection apparatus and the blood pressure compensation value

S1102

FIG. 11

1202

1201

1203

| Sending module | Processing module | Receiving module |

FIG. 12

131

133

Processor

Communications interface

134

1300

Memory

132

FIG. 13

## INTERNATIONAL SEARCH REPORT

| International application No. |
| --- |
| **PCT/CN2020/085509** |

**A. CLASSIFICATION OF SUBJECT MATTER**

G06K 9/00(2006.01)i

According to International Patent Classification (IPC) or to both national classification and IPC

**B. FIELDS SEARCHED**

Minimum documentation searched (classification system followed by classification symbols)

G06K, A61B

Documentation searched other than minimum documentation to the extent that such documents are included in the fields searched

Electronic data base consulted during the international search (name of data base and, where practicable, search terms used)

WPI, EPODOC, CNKI, CNPAT, IEEE: 血压, 测量, 监测, 面部特征, 脸部特征, 眼睛, 瞳孔, 坐标, 高度, 距离, 角度, 手机, 手环, 手表, 穿戴式, blood pressure, measur+, monitor, facial feature, eye, pupil, coordinate, height, distance, angle, cell phone, bracelet, watch, wearable

**C. DOCUMENTS CONSIDERED TO BE RELEVANT**

| Category* | Citation of document, with indication, where appropriate, of the relevant passages | Relevant to claim No. |
| --- | --- | --- |
| PX | CN 110222563 A (HUAWEI TECHNOLOGIES CO., LTD.) 10 September 2019 (2019-09-10) claims 1-29 | 1-29 |
| A | WO 2018083108 A1 (KONINKLIJKE PHILIPS N.V.) 11 May 2018 (2018-05-11) description, page 14, line 5 to page 20, line 25 | 1-29 |
| A | CN 101229058 A (THE CHINESE UNIVERSITY OF HONG KONG) 30 July 2008 (2008-07-30) entire document | 1-29 |
| A | CN 105748053 A (HUAWEI TECHNOLOGIES CO., LTD.) 13 July 2016 (2016-07-13) entire document | 1-29 |
| A | CN 105615859 A (ZHOU, Jialu) 01 June 2016 (2016-06-01) entire document | 1-29 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **23 June 2020** | **21 July 2020** |

| Name and mailing address of the ISA/CN | Authorized officer |
| --- | --- |
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/085509**

| Patent document cited in search report | | | Publication date (day/month/year) | Patent family member(s) | | | Publication date (day/month/year) |
|---|---|---|---|---|---|---|---|
| CN | 110222563 | A | 10 September 2019 | None | | | |
| WO | 2018083108 | A1 | 11 May 2018 | JP | 2020500052 | A | 09 January 2020 |
| | | | | EP | 3534785 | A1 | 11 September 2019 |
| | | | | CN | 109906052 | A | 18 June 2019 |
| | | | | US | 2019307339 | A1 | 10 October 2019 |
| CN | 101229058 | A | 30 July 2008 | None | | | |
| CN | 105748053 | A | 13 July 2016 | WO | 2017181635 | A1 | 26 October 2017 |
| CN | 105615859 | A | 01 June 2016 | EP | 3187106 | A1 | 05 July 2017 |
| | | | | WO | 2016029546 | A1 | 03 March 2016 |
| | | | | US | 2017290519 | A1 | 12 October 2017 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 201910343291